# EUROPEAN PATENT SPECIFICATION

(11) **EP 1 102 569 B1**
(45) Date of publication and mention of the grant of the patent: **17.09.2003**
(21) Application number: 99933429.5
(22) Date of filing: 02.07.1999
(51) Int. Cl.: A61F 13/15, A61L 15/24

(54) **USE OF A POLYETHENE MATERIAL PRODUCED FROM RENEWABLE RAW MATERIAL AS COMPONENT OF AN ABSORBENT ARTICLE**
BENUTZUNG EINES POLYETHENMATERIALS HERGESTELLT AUS ERNEUERBAREM ROHMATERIAL ALS KOMPONENTE EINES ABSORBIERENDEN ARTIKEL
UTILISATION DE MATIERE POLYETHENE PRODUITE A PARTIR D'UNE MATIERE PREMIERE RENOUVELABLE COMME COMPOSANT D'UN ARTICLE ABSORBANT

(30) Priority: 02.07.1998 SE 9802370
(43) Date of publication of application: 30.05.2001
(73) Proprietor: SCA Hygiene Products AB, 405 03 Göteborg (SE)
(72) Inventor: LAKSO, Elisabeth, S-444 46 Stenungsund (SE); SIMMONS, Eva, S-431 66 Mölndal (SE); NURMI, Hannele, S-471 42 Rönnäng (SE); JÖNBRINK, Anna, Karin, S-443 51 Lerum (SE); SILFVERSTRAND, Anders, S-435 44 Mölnlycke (SE)
(74) Representative: Bergstrand, Mikael Gudmundsson
(86) International application number: SE9901205
(87) International publication number: WO00001335

(56) References cited:
- EP-A1- 0 747 065
- GB-A- 587 378

## Description

The present invention relates to the use of material that contains polyethene produced from renewable raw material as a component of an absorbent article, absorbent articles, a method of producing an absorbent article, absorbent article components, and packaging material or units comprised of material that contains polyethene produced from renewable raw material.

Much thought is given to the care and protection of the environment in present-day societies. Newspapers, packaging materials comprised of glass, metal, paper, plastic, etc., are recycled with the purpose of conserving existing resources, such as oil, forest and metal. It is desirable to use materials that are as environmentally friendly as possible and that are reasonable in the manufacture of products. This is also very important with regard to the manufacture of sanitary and hygiene products for one-time use only, such as diapers, sanitary napkins, incontinence protectors or napkins, panty liners, etc., and with regard to the manufacture of packaging materials and packaging units. In addition to conserving our natural resources, it is also necessary to consider the environment with respect to the waste and contaminants to which it is subjected. Waste materials are dumped in garbage tips, where they are kept and, in the long term, break down or are alternatively incinerated. When we use so-called disposable articles and disposable packaging and wrapping materials, these articles and materials also land-up in garbage tips, which expand in size or are incinerated and therewith generate contaminants and carbon dioxide (CO₂). This contributes to the undesired greenhouse effect and to the consumption of natural resources.

Part of the community's resources is based on plants (Plantea) that continuously reproduce. Other resources exist in limited quantities and are regenerated very slowly. Petroleum products are an example of such resources. The use of petroleum raw materials depletes existing resources available to the community. It has taken many years for the oil that we use today to form.

The object of the present invention is to assist in alleviating these problems and to provide an absorbent disposable product and a packaging material that is more environmentally friendly than their known counterparts.

This object is achieved in accordance with the invention by the use of a material which contains polyethene and which is produced from renewable raw material. By renewable raw material is meant here a material produced from plant-based material. The renewable raw material is produced by plant material. Plants can be renewed by planting new trees, new potatoes, sowing new seed, etc. The opposite of a product produced from a renewable raw material is a product which consumes raw material that cannot be renewed, for instance polyethene produced from petroleum raw products. In TNC's Energy dictionary, a renewable energy source is defined as an energy source that can be reproduced at the same rate as it is used. Examples of renewable energy sources are forest energy, energy forests and energy crops. The same significance of the term renewable is used here, even though it is not an energy source that is concerned but a raw material.

There are some examples of what some people consider to be environmentally friendly absorbent articles. One example is products that can be used several times, by washing the products between use. Cloth diapers are used in this way.

US-A-5,032,119 teaches a reusable cloth diaper. Environmentally friendly disposable products can be products that comprise components produced from degradable material, such as polycaprolactone, polylactide or latex material. WO-A1-9407941 teaches a film produced from polylactide, which is biodegradable and can be composted and which can be used in diapers, for instance. Another degradable material that can be used in films comprises copolymers that include polycaprolactone and polylactide blocks, such material being described in WO-A1-9529200. This film can be used in diapers, for instance. Biodegradable latex material is used as film in diapers, as described in EP-A1-454 104. Polylactide is an example of renewable material that is used in absorbent articles. Starch, which is a renewable material, is also used in combination with polycaprolactone.

The invention relates to the use of material that contains polyethene produced from renewable raw material, as a component of an absorbent article, such as a diaper, sanitary napkin, incontinence protector, panty liner, a pant diaper or like articles.

The invention also relates to a method of producing an absorbent article, such as diaper, sanitary napkin, incontinence protector, panty liner, pant diaper or like article.

Finally, the invention also relates to the use of a packaging material that includes film that consists of a material which includes polyethene that has been produced from renewable raw material.

The components of the absorbent articles in question are all those that can be produced from polyethene, and also other conceivable components that may possibly be produced from polyethene. Examples of components produced from polyethene are plastic sheets that function as liquid impermeable backing sheets on absorbent articles, waist elastic in diapers for instance, top sheets on sanitary napkins and panty liners for instance, and tape used as diaper fastening means.

Part of a packaging unit may comprise film material that includes polyethene. When the packaging unit is comprised of several parts, it is not necessary for all of these parts to consist of said material, but they may include another type of plastic film or some other suitable material, for instance. The aforesaid packaging part may also have a form other than film in packaging units that can conceivably be produced from polyethene.

Polyethene is at present produced by polymerisation of ethene obtained by thermal (vapour) and catalytic cracking of different hydrocarbons, all from ethane derived from natural gas to crude oil.

The production of polyethene will now be described, such production being described in Textbook of Polymer Science, Third Edition, Fred W. Billmeyer, JR, A. Wiley-Interscience Publication John Wiley & Sons.

Ethene can be polymerised with benzene or chlorobenzene as a solvent. Both polymer and monomer in these compounds dissolve at the temperatures and pressures used, such that the reactions are purely solvent polymerisations. Water or other liquids can be added to drainoff reaction heat.

In continuous processes, there are used tubular reactors which may have diameters smaller than 2.5 cm and lengths of up to 30 m. The stainless steel pipe is filled with water, and ethene containing initiator and possibly benzene is introduced. Additional initiator and water or benzene can be injected into the system at one or more points along the pipe, or tube, so as to maintain the initiator concentration essentially constant through the reactor. Ten percent, or a higher percentage, of ethene is polymerised at the distal end of the reactor. The gas and liquid phases are continuously removed at this point and the polymer separated out. The ethene that remains is recycled, subsequent to being purified.

Another process uses bulk polymerisation in a tower-type reactor. Ethene containing trace quantities of oxygen is introduced into the reactor at 1500 atm and 190°C. The reaction is kept essentially isothermic and is carried out to a yield of 10-15%. The reactor outlet passes to a separation vessel in which unconverted ethene is removed for recycling. The molten polyethene is cooled to a temperature beneath its crystalline melting point and passed through the usual terminating stages.

LDPE (low density polyethene) can be produced in the aforedescribed way, this polyethene being the polyethene used primarily in the manufacture of polyethene film.

HDPE (high density polyethene), which can also be used to produce film, can be manufactured in several ways, including radical polymerisation of ethene at extremely high pressures, coordination polymerisation of ethene, and polymerisation of ethene supported by metal oxide catalysts.

In coordination polymerisation of ethene, there is used a catalyst produced as a colloidal dispersion by reacting alkyl aluminium and TiCl₄ in a solvent, such as heptane. Ethene is introduced into the reaction vessel under a weak pressure and at a temperature of 50-75°C. Polymerisation heat is removed by cooling. The polymer is produced in a powder or granule form, insoluble in the reaction mixture. The catalyst is destroyed at the end of the reaction process, by allowing water or alcohol to enter the system, and the polymer is filtered or centrifuged off, washed and dried.

Supportive metal oxide catalysts can be used in different working processes, including solid beds, moveable beds, fluidized beds and slurry processes. Ethene is supplied with a paraffin or cycloparaffin as an extender, at 60-200°C and a pressure of about 3.5 kPa. The polymer is recovered by cooling, or by solvent evaporation.

In the same process as that used to produce a HDPE, a polyethene having a certain degree of elasticity can be produced. In this case, there is used a metallocene catalyst and a small amount of some other monomer is added, such as hexene or butene.

Thus, at present ethene is taken from petroleum crude products, which are not renewable and which deplete natural resources in this respect. Furthermore, the incineration of polyethene results in the forming of carbon dioxide, which contributes to the undesired greenhouse effect.

According to the invention, renewable ethene is used to produce an environmentally friendly product, where the ethene is produced from a renewable raw material, such as ethanol. Ethanol is renewable when it is produced from a reproducible plant (*Plantae*). Sugar is converted to ethanol and carbon dioxide by fermentation under the influence of yeast fungi: C₆H₁₂O₆→2 C₂H₅OH + 2 CO. Potatoes, seed, forest raw materials or other plants are used in the fermentation process. Every fruit, berry or plant constituent that includes sugar can be fermented.

Ethene is produced from the renewable ethanol, by dehydrating ethanol for instance. Alcohol loses a water molecule and forms alkene when heated with a strong acid. Ethanol is heated to 180°C with concentrated sulphuric acid:
C₂H₅OH (conc, H₂SO₄, 180°C)→C₂H₄ + H₂O

Polyethene can be produced from the renewable ethene in the aforedescribed manner, already known in the art. It is also known to produce ethene from ethanol in the manner described above. The novelty in the present context resides in the use of renewable raw materials in the manufacture of polyethene for use in absorbent articles, which according to the invention results in environmentally friendly absorbent articles. Polyethene is used as material in components of the article, for instance as liquid-impermeable backing sheets, outer sheets or top sheets, diaper fastening tape, or as waist elastic. The novelty also resides in the use of renewable raw materials in manufacturing polyethene for use as packaging material.

An alternative to ethene produced from renewable ethanol is "cracking" of long carbon chains to ethene, such as the carbon chains of oils and fats. In this process, the long carbon chains of oils and fats are broken down to smaller molecules, of which some are ethene molecules. Naturally, in order to be renewable the oils and fats will be vegetable oil and fats. Many compounds can also be reacted to form ethene via ethanol, for instance acetic acid and ethylene oxide.

The invention relates to an entirely novel use of material that contains polyethene produced from renewable raw materials in absorbent articles and packaging materials.

It is not known in an industrial scale, to use polyethene that has been produced from renewable raw materials for the manufacture of environmentally friendly absorbent articles and environmentally friendly packaging materials which represent a lessening of the load on our environment and which do not deplete existing petroleum resources. Another advantage afforded by the invention is found in the possible incineration of disposable products and disposable packaging materials subsequent to their use. Incineration of polyethene generates carbon dioxide. This carbon dioxide contributes to the undesired greenhouse effect. When using renewable raw materials, however, CO₂ is consumed in the formation of the plants. This positive effect is also obtained when the products or packaging materials are dumped on the garbage or rubbish tip, since CO₂ has also been consumed in the formation of the plants in this case. The use of renewable raw materials thus has a mitigating effect on the greenhouse effect.

The invention will now be described in more detail with reference to the accompanying drawing, in which
Figure 1 is a sectioned view of an absorbent article, such as a diaper;
Figure 2 shows a diaper from above;
Figure 3 is a side view of an absorbent article packaged in polyethene film; and
Figure 4 is a side view showing several absorbent articles packaged in polyethene film.

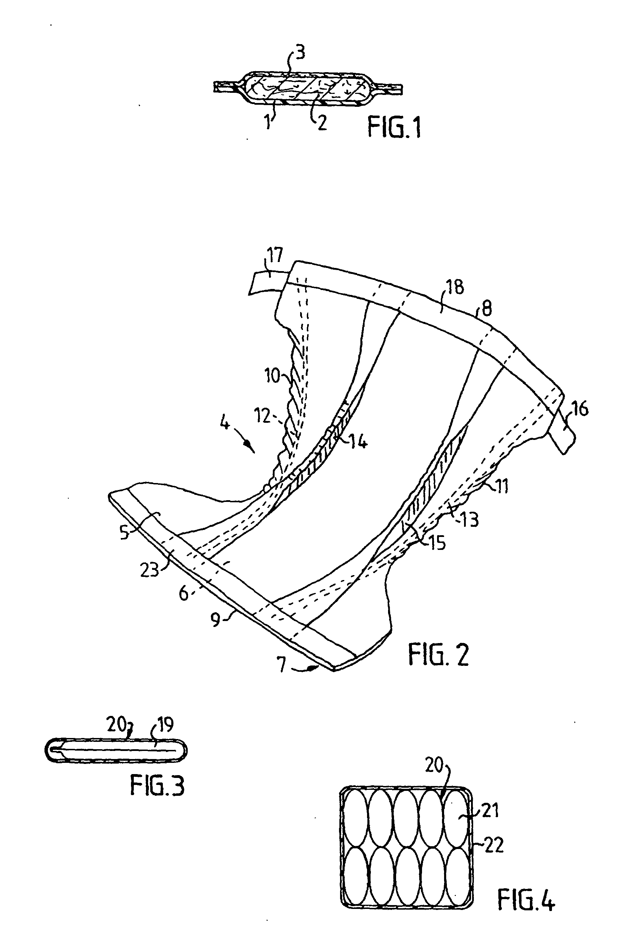

Polyethene is produced from renewable raw material, processed and then used as components of an absorbent article, such as a diaper, sanitary napkin, incontinence protector, panty liner, pant diaper or like article. The polyethene produced from renewable raw materials is also used for packaging material components. The packaging components concerned are, for instance, film or some other part of a packaging unit. Fig. 1 is a sectioned view of an absorbent article, which may be a diaper or a sanitary napkin, and Fig. 2 illustrates by way of example an absorbent article in the form of a diaper. The absorbent article in Fig. 1 includes a bottom liquid-impermeable barrier sheet 1, which in this document is referred to as a liquid-impermeable backing sheet 1 , an absorbent layer 2, and a top liquid-permeable outer sheet or surface sheet 3 which is intended to lie proximal to the wearer in use.

Fig. 2 illustrates a diaper 4 that includes a top liquid-permeable sheet 5, an absorbent sheet or unit 6, and a bottom liquid-impermeable backing sheet 7, said sheets being delimited by two transverse edges 8, 9 and two longitudinal edges 10, 11. The diaper also includes longitudinally extending leg elastic 12, 13 and possibly a liquid barrier 14, 15 on each side of the longitudinal centre line. The diaper also includes fastening devices in the form of fastener tapes 16, 17 and waist elastic 18, 23. The polyethene is used as component material in the liquid-impermeable backing sheet, waist elastic, top sheet, and fastener tape. Even other components may conceivably be produced from material that contains polyethene. The liquid-impermeable backing sheet 1, 7 is the sheet that prevents liquid leaking from the article. In the case of sanitary napkins and panty liners, the top sheet 3, 5 may also be produced from polyethene. The outer sheet or top sheet 3, 5 is the sheet that is uppermost and lies proximal to the wearer in use. This sheet shall be permeable to liquid, so that discharged liquid can be quickly drawn by suction down into the underlying absorbent sheet 2, 6. Diapers also include waist elastic 18, 23 and fastener devices 16, 17 in the form of tape. The waist elastic 18, 23 is positioned on the diaper in waist-height to make the diaper flexible and comfortable for the wearer in use and the fastener devices 16, 17 in the form of adhesive tape or in the form of touch-and-close fasteners by means of which the diaper can be secured in use so as not to loosen from the wearer.

By way of example of an absorbent article, Fig. 3 shows a folded sanitary napkin 19 enclosed in a packaging unit 20 comprised of film that includes polyethene produced from renewable raw material and Fig. 4 shows several sanitary napkins 21 wrapped in respective packaging material 21 which comprises film that contains polyethene produced from renewable raw material, said individual packets being enclosed in a packaging unit 22 comprising film material that includes polyethene produced from renewable raw material. The absorbent articles in the packages may include components comprised of material that includes polyethene produced from renewable raw material, although absorbent articles that include components made of completely different materials may also be included. In the packaging method illustrated in Fig. 4, one of the packages (21, 22) may consist of film material that includes polyethene produced from renewable raw material, while the other packages may consist of a completely different material.

The invention thus relates to the use of a material that contains polyethene produced from renewable raw material as a component of an absorbent article, such as a diaper, sanitary napkin, incontinence protector, panty liner, pant diaper or the like.

The material used may comprise up to 100% polyethene that has been produced from renewable raw material. Alternatively, the polyethene may be mixed with other materials, such as starch, for facilitating degradation of the material, for instance. Many different materials can be used together with the polyethene. Examples include other renewable materials, non-renewable materials or fillers. When the material used contains polyethene produced from renewable raw material and also contains some other material, the polyethene may be present in an amount corresponding to about 50 to 99% and the remainder consisting of some other material. The percentile proportion of said other material will depend on the nature of the material and the reason why it has been mixed with the polyethene. In respect to relatively large percentages of polyethene, the polyethene may be present in quantities corresponding to 60-80%. At times, only a small percentage of this other material will be used, e.g. percentages of 5% or from 1 to 20%, for instance, in which case the polyethene produced from renewable raw material will be 95% or from 80 to 99%. A feasible material mixture is one in which there is used polyethene produced from renewable raw material and polyethene produced from a petroleum product. The proportion of polyethene produced from renewable raw material will vary from 1 to 99%. Thus, the percentage of polyethene produced from renewable raw material will depend on the purpose and on the material mixed therewith. The material described above is also included in a following Claim as a mixture. When the polyethene produced from renewable raw material is mixed with some other material, this is also referred to as a mixture. The material composition described here also applies to the material used as packaging in accordance with the invention.

The components used in the absorbent articles are produced in accordance with known technology. Film can be produced and used in the manufacture of liquid-impermeable backing sheets which are then included in the diaper manufacturing process, this process also being carried out in accordance with conventional methods. Film can also be used as tape for the fastener devices. Top sheets and waist elastic are also produced in a conventional manner and included in the conventional manufacture of absorbent articles. For instance, top sheets may be made of film and then perforated. Surface material can also be produced in the form of nonwoven, by carding fibres that are then bonded in ovens. However, this is a question of bicomponent fibres of polyethene/polypropene. In the case of metallocene catalysts, elastic polyethene material can be produced for use, e.g., in waist elastic subsequent to having produced film from said material. As before mentioned, the components may be, e.g., backing sheets, i.e. liquid-impermeable sheets, found in all types of absorbent articles, top sheets found in, e.g., sanitary napkins and panty liners, waist elastic in diapers and fastener devices found primarily in diapers. The components recited in the depending Claims will thus be contingent on the type of article concerned in each respective case.

The invention also relates to a method for producing an absorbent article such as a diaper, sanitary napkin, incontinence protector, panty liner, pant diaper or the like, in which ethene is produced from renewable raw material, preferably ethanol, and polymerised to polyethene, wherein a film containing polyethene is obtained, by forming at least one article component from said film, and by feeding the component into a machine together with an absorbent body or pad and possibly remaining sheets, and joining the components together to form an absorbent article.

An absorbent article will normally include a bottom liquid-impermeable barrier sheet, an absorbent sheet on top of said liquid-impermeable backing sheet, a top liquid-permeable outer sheet which is intended to lie proximal to the wearer in use, waist elastic and fastener devices.

A life-cycle analysis (LCA) comprises the stages included in the aforesaid method and also in the use of the absorbent article and the recovery of the used article. In the article recovery process, the article is broken down or incinerated. Carbon dioxide generated during combustion or degradation and in the production of ethanol is consumed in corresponding quantities in the new formation of raw materials, such as potatoes, seed and trees, for instance.

Ethanol is produced from a plant in a conventional manner and ethene is produced from the ethanol as described above. The ethene is then polymerised to polyethene, which has also been described above. The components to be included in the absorbent article are then produced. The component produced may be film for use in producing the liquid-impermeable backing sheet of an article. Film may be produced by a film blowing process, a moulding process, or by cold roll extrusion. The film is then introduced into the article manufacturing process in a conventional manner in which the film is applied to the article, for instance in a diaper manufacturing machine. Alternatively, the component can be produced in some other way, for instance as components for use as top sheets described above. Subsequent to its manufacture, the component is introduced into the article production line.

An absorbent article component, said component being, for instance, a liquid-impermeable backing sheet 1, 7, a top sheet 3, 5, fastener means 16, 17, or waist elastic 18, 23 can be comprised of a material which includes polyethene, where at least a part of the polyethene is produced from renewable raw material, preferably ethene produced from ethanol.

An absorbent article, such as a diaper, sanitary napkin, incontinence guard, panty liner, pant diaper can have therefore at least one component comprised of a material that contains polyethene that has been produced from a renewable raw material, preferably ethene produced from ethanol.

The articles will normally include a bottom liquid-impermeable backing sheet 1, 7, an absorbent sheet or absorbent unit 2, 6 which lies on said sheet, a top or upper liquid-permeable outer sheet 3, 5, fastener means 16, 17 and waist elastic 18, 23.

These absorbent articles 4, 19, 21 can be packed individually, as at 19, or as indicated at 20, 22, several articles 21 may be packed and packaged in polyethene film produced from renewable raw material, preferably ethene produced from ethanol. When the absorbent articles 19, 21 are packaged in a larger, multi-pack unit 22, they may already be enclosed in individual packets 19 or may lack such packeting. Prepacking and the manufacture of the prepack or package are effected in accordance with known methods.

The absorbent articles can be packaged individually as at 19 or several articles 21 can be enclosed in a packaging unit 20, 22 comprised of film that contains polyethene produced from renewable raw material, preferably ethene produced from ethanol.

A packaging unit 20, 22 can include film that is comprised of material which includes polyethene produced from renewable raw material, preferably ethene produced from ethanol. In this case, as in all other embodiments of the invention, the material may consist entirely of polyethene produced from renewable raw material, or may comprise material that includes 50-99% polyethene. The examples of material and percentages mentioned above also apply to the packaging material.

The package is produced in accordance with conventional methods. For instance, film can be produced from the material that includes polyethene and a package then produced. As before mentioned, the packaging material component need not consist solely of film, but may also include some other component.

The packaging material including polyethene film produced from renewable raw material can be used in any selected type of packaging, preferably packaging of an absorbent product, such as a diaper, a sanitary napkin, an incontinence protector, a panty liner, pant diaper or the like. However, the packaging may also concern paper wipes, for instance kitchen paper, toilet paper, cloth wipes and the like. Thus, the packaged product need not always consist of a product that includes a component containing polyethene produced from renewable raw material. Neither need the packaged article be an absorbent product.

A material that contains polyethene produced from renewable raw material can be used to package different products.

One advantage afforded by the invention is that it is environmentally friendly by virtue of including components that are produced from material which contains polyethene and which, in turn, is produced from renewable raw material. This raw material does not deplete the petroleum sources of a community. Another advantage afforded by the use of renewable raw materials is that plants consume carbon dioxide as they develop, meaning that the greenhouse effect will not increase when using renewable raw materials instead of petroleum raw materials when said products are incinerated after use. This advantage also applies if the product is not incinerated, since the plant has already absorbed CO₂ and therewith contributed to a reduction in the greenhouse effect.

## Claims

1. The use of a material that includes polyethene produced from renewable raw material as a component of an absorbent article, such as a diaper, sanitary napkin, incontinence protector, panty liner, or pant diaper.

2. The use according to Claim 1, **characterised in that** the material consists of 100% of said polyethene.

3. The use according to Claim 1, **characterised in that** the material comprises from 50 to 99% of said polyethene.

4. The use according to Claim 1, **characterised in that** the component is a liquid impermeable backing sheet.

5. The use according to Claim 1, **characterised in that** the component is an outer sheet or top sheet.

6. The use according to Claim 1, **characterised in that** the component is a waist elastic.

7. The use according to Claim 1, **characterised in that** the component is a fastener device.

8. A method of producing an absorbent article such as a diaper, a sanitary napkin, an incontinence protector, a panty liner, or pant diaper producing ethene from renewable raw material, preferably ethanol, polymerising the ethene to polyethene, producing film containing said polyethene; forming at least one article component from said film, feeding said component into a machine together with an absorbent body and possibly other sheets, and joining said component to the absorbent article.

9. A method according to Claim 8, **characterised by** forming the film solely from said polyethene.

10. A method according to Claim 8, **characterised by** forming the film from a mixture that includes from 50 to 99% of said polyethene.

11. A method according to Claim 8, **characterised by** forming a liquid-impermeable backing sheet from said film.

12. A method according to Claim 8, **characterised by** forming an outer sheet or top sheet from said film.

13. A method according to Claim 8, **characterised by** forming waist elastic from said film.

14. A method according to Claim 8, **characterised by** forming a fastener device from said film.

15. The use of a film comprised of material that contains polyethene produced from renewable raw material, preferably ethene produced from ethanol, as a component of a packaging material.

16. Packaging material according to Claim 15, **characterised in that** the material consists in 100% of said polyethene.

17. Packaging material according to Claim 15, **characterised in that** said material comprises 50 to 99% of said polyethene.

## Patentansprüche

1. Verwendung eines Materials, welches Polyethen enthält, das aus einem erneuerbaren Rohstoff hergestellt wurde, als Bestandteil eines absorbierenden Gegenstands, wie einer Windel, einer Damenbinde, eines Inkontinzenzschutzes, einer Slipeinlage oder einer Hosenwindel.

2. Verwendung gemäss Anspruch 1, **dadurch gekennzeichnet, dass** der Stoff zu 100 % aus dem Polyethen besteht.

3. Verwendung gemäss Anspruch 1, **dadurch gekennzeichnet, dass** der Stoff 50 bis 99 % des Polyethens umfasst.

4. Verwendung gemäss Anspruch 1, **dadurch gekennzeichnet, dass** der Bestandteil eine flüssigkeitsundurchlässige Rückenlage ist.

5. Verwendung gemäss Anspruch 1, **dadurch gekennzeichnet, dass** der Bestandteil eine äussere Lage oder oberste Lage ist.

6. Verwendung gemäss Anspruch 1, **dadurch gekennzeichnet, dass** der Bestandteil ein Taillengummistoff ist.

7. Verwendung gemäss Anspruch 1, **dadurch gekennzeichnet, dass** der Bestandteil eine Befestigungsvorrichtung ist.

8. Verfahren zur Herstellung eines absorbierenden Gegenstands, wie einer Windel, einer Damenbinde, eines Inkontinenzschutzes, einer Slipeinlage oder einer Hosenwindel, umfassend das Herstellen von Ethen aus einem erneuerbaren Rohstoff, vorzugsweise Ethanol, das Polymerisieren des Ethens zu Polyethen, das Herstellen einer dieses Polyethen enthaltenden Folie; das Formen mindestens eines Bestandteils eines Gegenstands aus der Folie, das Einführen des Bestandteils in eine Maschine zusammen mit einem absorbierenden Körper und gegebenenfalls anderen Lagen, und das Verbinden des Bestandteils mit dem absorbierenden Gegenstand.

9. Verfahren gemäss Anspruch 8, **dadurch gekennzeichnet, dass** man die Folie ausschliesslich aus dem Polyethen formt.

10. Verfahren gemäss Anspruch 8, **dadurch gekennzeichnet, dass** man die Folie aus einer Mischung formt, die 50 bis 99 % des Polyethens enthält.

11. Verfahren gemäss Anspruch 8, **dadurch gekennzeichnet, dass** man eine flüssigkeitsundurchlässige Rückenlage aus der Folie formt.

12. Verfahren gemäss Anspruch 8, **dadurch gekennzeichnet, dass** man eine äussere Lage oder oberste Lage aus der Folie formt.

13. Verfahren gemäss Anspruch 8, **dadurch gekennzeichnet, dass** man einen Taillengummistoff aus der Folie formt.

14. Verfahren gemäss Anspruch 8, **dadurch gekennzeichnet, dass** man eine Befestigungsvorrichtung aus der Folie formt.

15. Verwendung einer Folie, die ein Material umfasst, das Polyethen enthält, welches aus einem erneuerbaren Rohstoff hergestellt wurde, vorzugsweise aus Ethanol hergestelltes Ethen, als Bestandteil eines Verpackungsmaterials.

16. Verpackungsmaterial gemäss Anspruch 15, **dadurch gekennzeichnet, dass** das Material zu 100 % aus dem Polyethen besteht.

17. Verpackungsmaterial gemäss Anspruch 15, **dadurch gekennzeichnet, dass** das Material 50 bis 99 % des Polyethens umfasst.

## Revendications

1. Utilisation d'un matériau qui comprend du polyéthène produit à partir de matière brute renouvelable comme composant d'un article absorbant tel qu'une couche-culotte, une serviette hygiénique, une protection d'incontinence, un protège-slip, ou un "pant diaper".

2. Utilisation selon la revendication 1, **caractérisée en ce que** le matériau consiste dans 100% dudit polyéthène.

3. Utilisation selon la revendication 1, **caractérisée en ce que** le matériau comprend de 50 à 99% dudit polyéthène.

4. Utilisation selon la revendication 1, **caractérisée en ce que** le composant est une feuille de soutien imperméable aux liquides.

5. Utilisation selon la revendication 1, **caractérisée en ce que** le composant est une feuille externe ou feuille supérieure.

6. Utilisation selon la revendication 1, **caractérisée en ce que** le composant est un élastique pour la taille.

7. Utilisation selon la revendication 1, **caractérisée. en ce que** le composant est un dispositif d'attache.

8. Procédé de production d'un article absorbant tel qu'une couche-culotte, une serviette hygiénique, une protection d'incontinence, un protége-slip, ou un "pant diaper", qui produit de l'éthène à partir d'une matière brute renouvelable, de préférence de l'éthanol, qui polymérise l'éthène en polyéthène en produisant un film contenant ledit polyéthène; formant au moins un composant d'article à partir dudit film, en alimentant ledit composant dans une machine avec un corps absorbant et éventuellement avec d'autres feuilles, et en joignant ledit composant à l'article absorbant.

9. Procédé selon la revendication 8, **caractérisé par** la formation du film à partir dudit polyéthène uniquement.

10. Procédé selon la revendication 8, **caractérisé par** la formation du film à partir d'un mélange qui contient de 50 à 99% dudit polyéthène.

11. Procédé selon la revendication 8, **caractérisé par** la formation d'une feuille de soutien imperméable aux liquides à partir dudit film.

12. Procédé selon la revendication 8, **caractérisé par** la formation d'une feuille externe, ou feuille supérieure, à partir dudit film.

13. Procédé selon la revendication 8, **caractérisé par** la formation d'élastique pour la taille à partir dudit film.

14. Procédé selon la revendication 8, **caractérisé par** la formation d'un dispositif d'attache à partir dudit film.

15. Utilisation d'un film constitué d'un matériau qui contient du polyéthène produit à partir de matière brute renouvelable, de préférence de l'éthène produit à partir d'éthanol, comme composant d'un matériau d'emballage.

16. Matériau d'emballage selon la revendication 15, **caractérisé en ce que** le matériau consiste dans 100% dudit polyéthène.

17. Matériau d'emballage selon la revendication 15, **caractérisé en ce que** le matériau comprend entre 50 et 99% dudit polyéthène.
